# EUROPEAN PATENT SPECIFICATION

(11) **EP 1 401 368 B1**
(45) Date of publication and mention of the grant of the patent: **08.03.2006**
(21) Application number: 02741599.1
(22) Date of filing: 26.06.2002
(51) Int. Cl.: A61F 13/62

(54) **A SANITARY ABSORBENT ARTICLE INCLUDING A FASTENER**
SAUGFÄHIGER HYGIENEARTIKEL MIT BEFESTIGUNGSMITTEL
ARTICLE ABSORBANT SANITAIRE A MOYENS DE FIXATION

(30) Priority: 28.06.2001 SE 0102308
(43) Date of publication of application: 31.03.2004
(73) Proprietor: SCA Hygiene Products AB, 405 03 Göteborg (SE)
(72) Inventor: BÄCK, Lucas, S-414 57 Göteborg (SE)
(74) Representative: Hyltner, Jan-Olof
(86) International application number: PCT/SE2002/001265
(87) International publication number: WO 2003/002053

(56) References cited:
- WO-A1-99/13745
- SE-C2- 504 624
- US-A- 5 053 028
- US-A- 5 722 968

## Description

### TECHNICAL AREA

The present invention relates to a sanitary, absorbent article including at least one hooks and loops type fastener for releasably attaching a first and a second part of the article to each other, wherein the first part is overlapping the second part when said parts are attached to each other, the fastener including a hooks member affixed to said first part of the article and a loops member affixed to said second part of the article, each member comprising a base portion having a bottom surface affixed to the first or second part of the article and an opposite top surface containing hooks elements and loops elements, respectively.

### BACKGROUND OF THE INVENTION

Fastener of the hooks and loops type are nowadays often used to releasably attach different parts of sanitary absorbent articles to each other, such as the front and rear side parts of a diaper or the end parts of a waist belt. When affixing the members of such fasteners to an irregular surface, such as the surface of a nonwoven material, delamination of the members have occurred in several cases when the articles containing the members have been subjected to loads corresponding to the loads during normal use of the article.

An object of the present invention is to eliminate or at least to a great extent reduce the risk for delamination of fasteners of the hooks and loops type.

### SUMMARY OF THE INVENTION

This object is achieved by a sanitary, absorbent article including a hooks and loops type fastener for releasably attaching a first and a second part of the article to each other, wherein the first part is overlapping the second part when said parts are attached to each other, the fastener includes a hooks member affixed to said first part of the article and a loops member affixed to said second part of the article, each member comprising a base portion having a bottom surface affixed to the first or second part of the article and an opposite top surface containing hooks elements and loops elements, respectively, the whole of the bottom surface of the base portion of the hooks or loops member is affixed to the first or second part, characterised in that the fastener has a first end region in which the engagement force between the hooks and loops element of the members is reduced. Thereby a reduction of the delamination force, i.e. the force component directed perpendicular to the plane of the second part of the article, is accomplished.

In a preferred embodiment the first end region of the fastener has a width of 3-8 mm, preferably 5 mm and the hooks member is without hooks elements in the first end region.

Alternatively the loops member is without loops elements in a portion thereof complementary to the first end region of the hooks member.

In a second embodiment the hook elements of the hooks member are made non-engageable with the loops elements of the loops member or vice versa in the portion of the fastener in which the first end region of the hooks member is disposed.

The hooks member may in both embodiments be affixed to a nonwoven material.

### BRIEF DESCRIPTION OF THE DRAWING

The present invention will now be described with reference to the following figures, of which;
Fig. 1 discloses schematically an incontinence garment according to a first embodiment of the invention in a perspective view,
Fig. 2 disclose a sectional view along line II-II of figure 1,
Fig. 3 and 4 disclose schematically the forces acting on a conventional hooks member of a fastener of the hooks and loops type in the middle in a side view and in a plan view, respectively,
Fig. 5 and 6 disclose schematically the forces acting on a conventional hooks member of a fastener of the hooks and loops type in the edge portion in a side view and in a plan view, respectively, and
Fig. 7 and 8 disclose schematically the forces acting on a hooks member according to an embodiment of the invention in a side view and a plan view, respectively.

### DESCRIPTION OF EMBODIMENTS

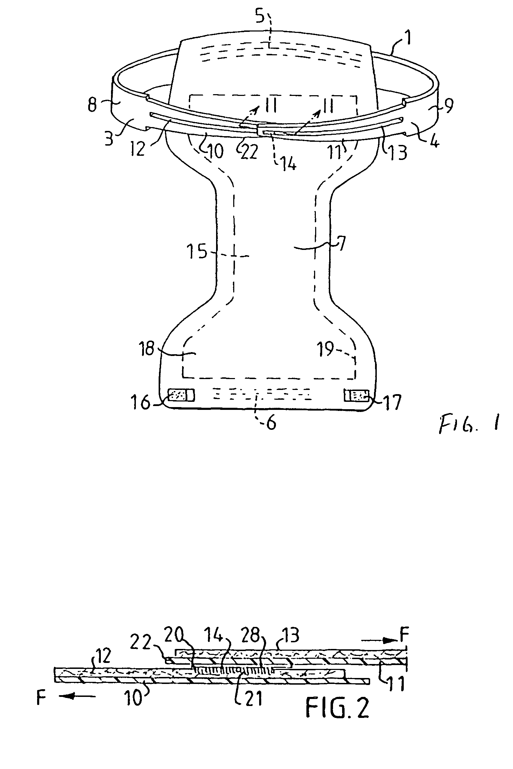

Figure 1 shows an incontinence garment comprising a waist belt 1 and an incontinence guard 7. The waist belt 1 illustrated in Figure 1 is made of a flexible material, preferably a nonwoven, and includes a rear portion 2 of the incontinence guard 7 and two front portions 3,4 affixed to the respective left and right side parts of the rear portion 2. The rear portion 2 comprises further an elastic waist element 5, for example elastic threads, which is attached to the rear portion 2 in a stretched state as is conventional for this type of article. The waist part of the front portion of the guard 7 is also made elastic in the same way by an elastic waist element 6.

The waist-belt front portions 3,4 include a relatively broad rear part 8 and 9 respectively, which connect with the rear portion and stretch over the hips of the wearer in use. Narrow, elongated and rectangular fastener members 12 and 13 extend along the longitudinal symmetry lines of the tapering portions 10,11, these fastener members preferably comprising loop-bearing material. A fastener member 14 complementary to the fastener member 12, preferably a piece of hook-bearing material, is attached to the inside of the front portion 11 at the end part thereof. Figure 1 shows the belt when fastened together, i.e. with the fastener member 14 in engagement with the fastener member 12. As will be understood, because the member 12 extends along substantially the full length of the tapering portion 10, the illustrated waist belt can be adjusted to fit around the waist of many users having mutually different waist sizes.

The incontinence guard 7 has in the front part thereof two fastener members 16,17 complementary to the fastener members 12,13 on the waist belt 1, the members 16, 17 preferably being pieces of hooks-bearing material. The incontinence guard comprises in a conventional manner an absorbent core 15 being enclosed between a liquid-permeable surface layer 18 and a liquid-impermeable surface layer 19, the members 16,17 being affixed to the liquid-permeable surface material 18. All materials known to be used for the components of a diaper or an incontinence guard can be used for the incontinence guard 7, such as a layer 18 of nonwoven, a body 15 of cellulose fluff pulp containing superabsorbent particles or not and a laminate 19 of a nonwoven and a vapour permeable film.

In Figure 2 a partial sectional view of the belt 1 is schematically shown, the portion 11 of the belt 1 overlapping the portion 10. The piece 14 of hooks-bearing material being affixed to the inside of the portion 11 is fastened to the elongate piece 12 of loops-bearing material. The forces acting on the belt portions 10 and 11 are indicated by arrows F in Figure 2.

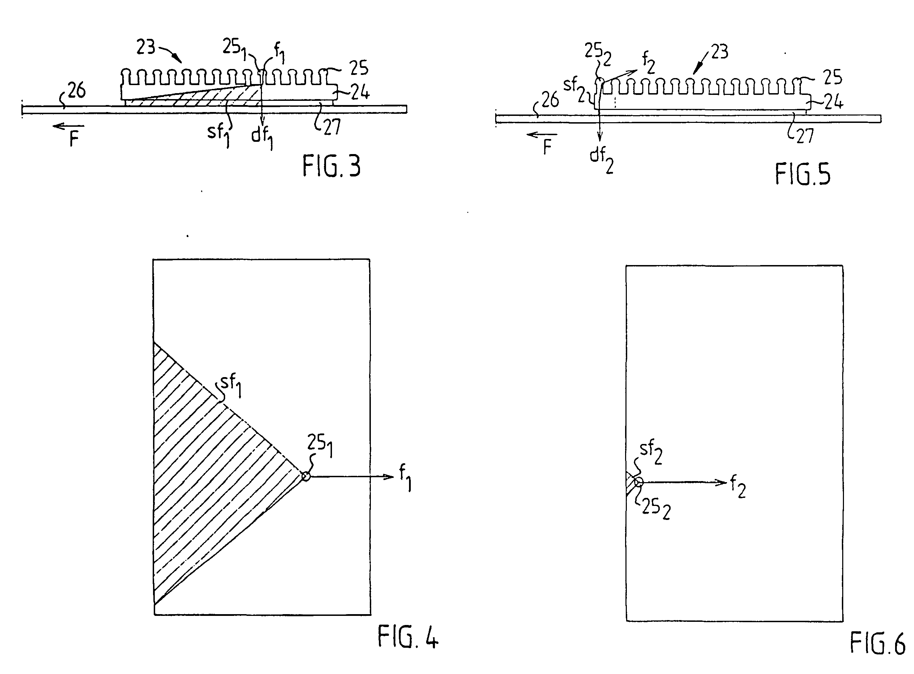

The piece 14 of hooks-bearing material consists of a base portion 20 having a bottom surface affixed to the inside of portion 11 of the belt 1 by gluing or heat welding and a top surface opposite from the bottom surface containing rows of hooks 21 protruding therefrom. In the disclosed embodiment, the end region of the base portion 20 being distal to the edge 22 of belt portion 11 is without protruding hooks in order to reduce the risk for delamination of the piece 14 from the belt portion 11 as will be explained below with reference to Figures 3-5. The pieces of hooks-bearing material affixed to the layer 18 in the front parts of the incontinence guard 7 are constructed in the same manner, the end regions thereof being distal to the respective left and right side edge of the incontinence guard 7 being without hooks, as schematically illustrated for the pieces 16,17 in Figure 1.

The risk for delamination of a piece of hooks- or loops-bearing material is due to a moment force acting on the base portion of said piece in an end region thereof. Every hook or loop of such pieces is distanced from the base portion thereof and the engagement point between a loop and a hook will therefore be distanced from the bottom of the base portion. In Figure 3 a piece 23 of hooks-bearing material having a base portion 24 and rows of hooks 25 protruding upwardly from the base portion, is schematically shown. The engagement force acting on a hook 25₁ when engaged with a loop (not shown for the sake of clarity) is represented by the arrow f₁. The large part of the force f₁ will be taken up in the base portion as shear forces, as indicated by the shade triangles sf₁ in Figures 3 and 4. Only a very little part of the force f₁ will act as a delamination force df₁ perpendicular to the plane of the base portion 24. However, as schematically shown in Figures 5 and 6, in a first end region of the piece 23 the force f₂ acting on a hook 25₂ can only to a small extent be taken up as shear forces sf₂ in the base portion 24 and the delamination force df₂ will be large compared to the force df₁ resulting from the force f₁ acting on the hook 25₁. The piece 23 is affixed to the layer 26 being for example a nonwoven material by an adhesive layer 27. The adhesive connection between the piece 23 and the layer 26 can withstand large shear forces but the delamination forces, i.e. forces perpendicular to the plane of base portion 24, required to delaminate the first end region of the piece 23 from the layer 26 are relatively small. It is therefore a risk that the delamination forces df₂ will be large enough to start a delamination of the piece 23 in the first end region thereof.

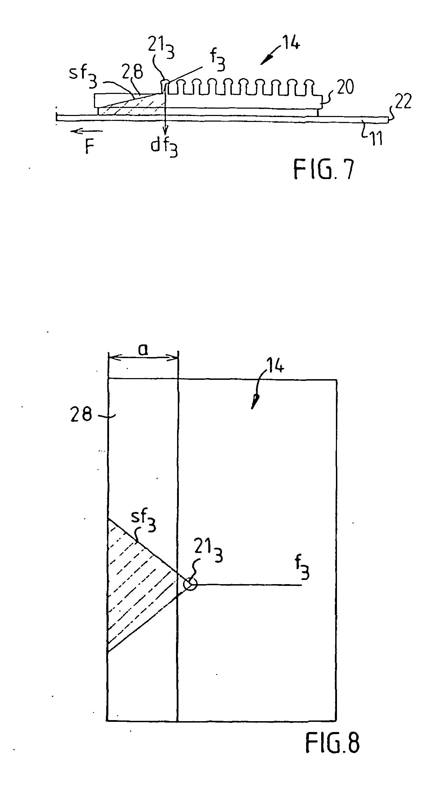

In Figures 7 and 8 the hooks member 14 affixed to the belt portion 11 is schematically shown. In order to reduce the risk for delamination, this hooks member has no hooks in a first end region 28 thereof. In these Figures, the reaction forces from a force f₃ acting on a hook 21₃ in the last row of hooks is schematically illustrated and it is evident that the force f₃ to the largest extent will be taken up as shear forces sf₃, only a small part being taken up as a delamination force df₃. The width a of the first end region 28 without hooks is 3-S mm, preferably around 5 mm.

The forces acting on a loops member will be analogous to the forces described above for the hooks member and the loops member can be constructed in the same way as the hooks member, i.e. without loops in a first end region thereof. It is pointed out that the first end region of a loops member lies opposite to the first end region of hooks member co-operating therewith. The first end of a hooks or loops member is the end lying distal from the overlapping edge of the element to which the respective member is attached, as seen in the direction of the forces. Thus, if the fastener is subjected to forces having different directions, the respective member can have more than one first end region. However, usually it is only the greatest force that need to be considered when the risk for delamination should be eliminated or reduced.

In the described embodiment the moment force created on a first end region of a hooks- and loops fastener has been reduced by providing a first end region of the hooks member without hooks. It is of course also possible to instead destroy the existing hooks in an end region of a hooks member in order to ensure that in the end region in question the hooks will not be able to engage the loops of a complementary loops member, for example by the application of heat and pressure if the hooks member is made of a heat deformable material or by cutting off the heads of the hooks.

The hooks members 16,17 on the front part of the incontinence guard 7 are preferable also provided with first end regions without hooks as schematically illustrated in Figure 1 for the members 16,17, the first end regions thereof lying distal from the respective left and right side edge of the incontinence guard 7. The gravity forces acting on the hooks members when the article is worn are smaller than the elastic forces and need not be considered.

Fasteners of the hooks and loops type having hooks or loops members with end regions in which the hooks or loops are not engageable to each other can of course be used for other articles than the absorbent garment shown in Figure 1. For example can a hooks member provided on a tab for a diaper be provided with such an end region, especially if the tab is made of a nonwoven material.

## Claims

1. A sanitary, absorbent article including a hooks and loops type fastener (14) for releasably attaching a first (11) and a second part (10) of the article to each other, wherein the first part (11) is overlapping the second part (10) when said parts are attached to each other, the fastener (14) includes a hooks member (20,21) affixed to said first part of the article and a loops member (12) affixed to said second part of the article, each member comprising a base portion (20) having a bottom surface affixed to the first or second part of the article and an opposite top surface containing hooks elements (21) and loops elements, respectively, the whole of the bottom surface of the base portion of the hooks or loops member is affixed to the first or second part, **characterised in that** the fastener (14) has a first end region (28) in which the engagement force between the hooks and loops element of the members is reduced.

2. The article according to Claim 1, **characterised in that** the first end region (28) of the fastener has a width of 3-8 mm, preferably 5 mm.

3. The article according to Claim 1 or 2, **characterised in that** the hooks member (20,21) is without hooks elements (21) in the first end region (28).

4. The article according to any one of Claims 1 or 2, **characterised in that** the loops member is without loops elements in an end portion thereof complementary to the first end region of the hooks member.

5. The article according to Claim 1 or 2, **characterised in that** the hook elements of the hooks member are made non-engageable with the loops elements of the loops member or vice versa in the portion of the fastener in which the first end region of the hooks member is disposed.

6. The article according to one of Claims 1-5, **characterised in that** the hooks member is affixed to a nonwoven material.

## Revendications

1. Un article absorbant, hygiénique comprenant une fixation du genre à crochets et à boucles (14) pour fixer de manière détachable une première (11) et une deuxième partie (10) de l'article l'une à l'autre, dans lequel la première partie (11) chevauche la deuxième partie (10) lorsque lesdites parties sont fixées l'une à l'autre, la fixation (14) comprend un membre à crochets (20, 21) appliqué sur la première partie de l'article et un membre à boucles (12) appliqué sur la deuxième partie de l'article, chaque membre comprenant une portion de base (20) ayant une surface inférieure appliquée sur la première ou deuxième partie de l'article et une surface supérieure opposée comprenant respectivement les éléments à crochets (21) et éléments à boucles, dans lequel la surface inférieure entière de la portion de base du membre à crochets ou à boucles est appliquée sur la première ou deuxième partie, **caractérisé en ce que** la fixation (14) présente une première région d'extrémité (28) dans laquelle la force de raccordement entre l'élément à crochets et à boucles des membres est réduite.

2. L'article selon la revendication 1, **caractérisé en ce que** la première région d'extrémité (28) de la fixation présente une largeur de 3-8 mm, préférablement 5 mm.

3. L'article selon la revendication 1 ou 2, **caractérisé en ce que** le membre à crochets (20, 21) ne présente pas d'éléments à crochets (21) dans la première région d'extrémité (28).

4. L'article selon l'une quelconque des revendications 1 ou 2, **caractérisé en ce que** le membre à boucles ne présente pas d'éléments à boucles dans sa portion d'extrémité qui est complémentaire à la première région d'extrémité du membre à crochets.

5. L'article selon la revendication 1 ou 2, **caractérisé en ce que** les éléments à crochets du membre à crochets sont faits de façon à être non raccordables avec les éléments à boucles des membres à boucles ou vice versa dans la portion de la fixation dans laquelle la première région d'extrémité du membre à crochets est disposée.

6. L'article selon l'une quelconque des revendications 1-5, **caractérisé en ce que** le membre à crochets est appliqué sur une matière non tissée.

## Patentansprüche

1. Absorbierender Hygieneartikel, umfassend einen Klettverschluss (14) zum lösbaren Anbringen eines ersten (11) und eines zweiten Teils (10) des Artikels aneinander, wobei der erste Teil (11) den zweiten Teil (10) überlappt, wenn die Teile aneinander angebracht sind, der Verschluss (14) ein Hakenelement (20, 21), das an dem ersten Teil des Artikels befestigt ist und ein Schlaufenelement (12), das an dem zweiten Teil des Artikels befestigt ist, umfasst, jedes Element einen Basisabschnitt (20) mit einer an dem ersten oder zweiten Teil des Artikels befestigten Boden und einer entgegengesetzten oberen Seite mit Haken (21) bzw. Schlaufen umfasst, wobei der gesamte Boden des Basisabschnitts des Haken- oder Schlaufenelements an dem ersten oder zweiten Teil befestigt ist, **dadurch gekennzeichnet, dass** der Verschluss (14) einen ersten Endbereich (28) aufweist, in dem die Eingriffskraft zwischen den Haken und Schlaufen der Elemente reduziert ist.

2. Artikel nach Anspruch 1, **dadurch gekennzeichnet, dass** der erste Endbereich (28) des Verschlusses eine Breite von 3-8 mm, vorzugsweise 5 mm, aufweist.

3. Artikel nach Anspruch 1 oder 2, **dadurch gekennzeichnet, dass** das Hakenelement (20, 21) in dem ersten Endbereich (28) keine Haken (21) aufweist.

4. Artikel nach einem der Ansprüche 1 oder 2, **dadurch gekennzeichnet, dass** das Schlaufenelement in einem Endabschnitt komplementär zu dem ersten Endbereich des Hakenelements keine Schlaufen aufweist.

5. Artikel nach Anspruch 1 oder 2, **dadurch gekennzeichnet, dass** die Haken des Hakenelements im Abschnitt des Verschlusses, in dem der erste Endbereich des Hakenelements angeordnet ist, derart ausgestaltet sind, dass sie mit den Schlaufen des Schlaufenelements nicht in Eingriff bringbar sind oder umgekehrt.

6. Artikel nach einem der Ansprüche 1 bis 5, **dadurch gekennzeichnet, dass** das Hakenelement an einem Vliesstoff befestigt ist.
